# EUROPÄISCHE PATENTSCHRIFT

(11) **EP 1 701 928 B1**
(45) Veröffentlichungstag und Bekanntmachung des Hinweises auf die Patenterteilung: **23.04.2008**
(21) Anmeldenummer: 04804419.2
(22) Anmeldetag: 30.12.2004
(51) Int. Cl.: C07C 5/333, C07C 11/167

(54) **VERFAHREN ZUR HERSTELLUNG VON BUTADIEN**
METHOD FOR THE PRODUCTION OF BUTADIENE
PROCEDE DE PRODUCTION DE BUTADIENE

(30) Priorität: 30.12.2003 DE 10361822
(43) Veröffentlichungstag der Anmeldung: 20.09.2006
(73) Patentinhaber: BASF SE, 67056 Ludwigshafen (DE)
(72) Erfinder: JOHANN, Thorsten, 67117 Limburgerhof (DE); SCHINDLER, Götz-Peter, 68219 Mannheim (DE); BRODHAGEN, Andreas, 67125 Dannstadt-Schauernheim (DE); CRONE, Sven, 67117 Limburgerhof (DE); DUDA, Mark, 67071 Ludwigshafen (DE)
(74) Vertreter: Isenbruck, Günter
(86) Internationale Anmeldenummer: PCT/EP2004/014834
(87) Internationale Veröffentlichungsnummer: WO 2005/063656

(56) Entgegenhaltungen:
- WO-A-20/04007408
- GB-A- 628 686

## Beschreibung

Die Erfindung betrifft ein Verfahren zur Herstellung von Butadien.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (Acrylnitril-Butadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein KohlenwasserstoffGemisch aus Methan, Ethan, Ethen, Acetylen, Propan, Propen, Propin, Allen, Butenen, Butadien, Butinen, Methylallen, C₅- und höheren Kohlenwasserstoffen an.

Nachteilig an der Erzeugung von Butadien im Crackprozess ist, dass zwangsläufig größere Mengen an unerwünschten Koppelprodukten anfallen.

Aufgabe der Erfindung ist es, ein Verfahren zur Herstellung von Butadien aus n-Butan bereitzustellen, bei dem in möglichst geringem Umfang Koppelprodukte anfallen.

Gelöst wird die Aufgabe durch ein Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative katalytische Dehydrierung von n-Butan, wobei ein Produktgasstrom b enthaltend n-Butan, 1-Buten, 2-Buten, Butadien, Wasserstoff, leichtsiedende Nebenbestandteile und gegebenenfalls Wasserdampf erhalten wird;
C) Einspeisung des Produktgasstroms b der nicht-oxidativen katalytischen Dehydrierung und eines sauerstoffhaltigen Gases in mindestens eine zweite Dehydrierzone und oxidative Dehydrierung von 1-Buten und 2-Buten, wobei ein Produktgasstrom c enthaltend n-Butan, 2-Buten, Butadien, Wasserstoff, leichtsiedende Nebenbestandteile und Wasserdampf erhalten wird, welcher einen höheren Gehalt an Butadien als der Produktgasstrom b aufweist;
D) Abtrennung von Wasserstoff, leichtsiedenden Nebenbestandteile und von Wasserdampf, wobei ein C₄-Produktgasstrom d im Wesentlichen bestehend aus n-Butan, 2-Buten und Butadien erhalten wird;
E) Einspeisung des C₄-Produktgasstroms d in eine Destillationszone und Abtrennung eines Butadien/n-Butan-Gemischs als Wertproduktstrom e1, wobei ein Strom e2 im Wesentlichen bestehend aus n-Butan und 2-Buten verbleibt;
F) Rückführung des Stroms e2 in die erste Dehydrierzone.

Das erfindungsgemäße Verfahren zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. Durch die Kopplung von nicht-oxidativer katalytischer Dehydrierung und oxidativer Dehydrierung wird eine hohe Butadien-Ausbeute erzielt.

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen gesättigte C₂-C₅-Kohlenwasserstoffe. Daneben enthält es auch Methan und Spuren von C₆⁺-Kohlenwasserstoffen. Die Zusammensetzung von LPG kann stark schwanken. Vorteilhafter Weise enthält das eingesetzte LPG mindestens 10 Gew.-% Butane.

Alternativ kann ein veredelter C₄-Strom aus Crackern oder Raffinerien eingesetzt werden.

In einer Variante des erfindungsgemäßen Verfahrens umfasst die Bereitstellung des n-Butan enthaltenden Dehydrier-Einsatzgasstromes die Schritte
(A1) Bereitstellung eines liquefied petroleum gas (LPG)-Stroms,
(A2) Abtrennung von Propan und gegebenenfalls Methan, Ethan und C₅⁺-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
(A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom wird vorzugsweise einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether eingesetzt werden.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden.

Ein Merkmal der nicht-oxidativen Fahrweise gegenüber einer oxidativen Fahrweise ist das Vorhandensein von Wasserstoff im Austragsgas. Bei der oxidativen Dehydrierung wird kein freier Wasserstoff in wesentlichen Mengen gebildet.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, GB 628686, US 4,996,387 und US 5,389,342). Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff und/oder Kohlenwasserstoffen in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne Sauerstoffhaltigem Gas als Co-Feed in einem Hordenreaktor durchgeführt werden. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauschertlächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff mit Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab.

Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stelle des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispiel 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaitigen Gas reduziert.

Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 1 bis 15 Vol.-% 1-Buten, 1 bis 25 Vol.-% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 70 Vol.-% Stickstoff und 0 bis 5 Vol.-% Kohlenstoffoxide.

Der die erste Dehydrierzone verlassende Produktgasstrom b wird bevorzugt in zwei Teilströme aufgetrennt, wobei nur einer der beiden Teilströme den weiteren Verfahrensteilen C bis F unterworfen wird und der zweite Teilstrom in die erste Dehydrierzone zurückgeführt werden kann. Eine entsprechende Verfahrensweise ist in DE-A 102 11 275 beschrieben. Es kann jedoch auch der gesamte Produktgasstrom b der nicht-oxidativen katalytischen n-Butan-Dehydrierung den weiteren Verfahrensteilen C bis F unterworfen werden.

Der nicht-oxidativen katalytischen Dehydrierung wird erfindungsgemäß eine oxidative Dehydrierung (Oxidehydrierung) als Verfahrensteil C nachgeschaltet. Dabei werden im Wesentlichen 1-Buten und 2-Buten zu 1,3-Butadien dehydriert, wobei 1-Buten im Allgemeinen nahezu vollständig abreagiert.

Diese kann grundsätzlich in allen aus dem Stand der Technik bekannten Reaktortypen und Fahrweisen durchgeführt werden, wie beispielsweise im Wirbelbett, im Hordenofen, im Festbettrohr- oder ―rohrbündeireaktor oder im Plattenwärmetauscherreaktor. Ein Plattenwärmetauscherreaktor ist beispielsweise in DE-A 199 52 964 beschrieben. Zur Durchführung der oxidativen Dehydrierung wird ein Gasgemisch benötigt, welches ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der nicht-oxidativen katalytischen Dehydrierung stammende Produktgasgemisch mit Sauerstoff oder einem sauerstoffhaltigem Gas, beispielsweise Luft, vermischt. Das erhaltene sauerstoffhaltige Gasgemisch wird dann der Oxidehydrierung zugeführt.

Die für die- Oxidehydrierung besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P₀,₅Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ)_{·}

Die Stöchiometrie der Aktivmasse einer Vielzahl der für die Oxidehydrierung geeigneten Multimelalloxidkatalysatoren lässt sich unter die allgemeine Formel (I)

Mo₁₂BiₐPe_{b}Co_{c}Ni_{d}Cr_{c}X¹_{f}K_{g}Oₓ (I),

in der die Variablen nachfolgende Bedeutung aufweisen:
- X¹ =: W, Sn, Mn, La, Ce, Ge, Ti, Zr, Hf, Nb, P, Si, Sb, Al, Cd und/oder Mg;
- a =: 0,5 bis 5, vorzugsweise 0,5 bis 2;
- b =: 0 bis 5, vorzugsweise 2 bis 4;
- c =: 0 bis 10, vorzugsweise 3 bis 10;
- d =: 0 bis 10;
- e =: 0 bis 10, vorzugsweise 0,1 bis 4;
- f =: 0 bis 5, vorzugsweise 0,1 bis 2;
- g =: 0 bis 2, vorzugsweise 0,01 bis 1; und
- x =: eine Zahl, die durch die Wertigkeit und Häufigkeit der von Sauerstoff verschiedenen Elemente in (I) bestimmt wird;
subsumieren.

Bevorzugt setzt man beim erfindungsgemäßen Verfahren für die Oxidehydrierung ein Mo-Bi-Fe-O-haltiges Multimetalloxidsystem ein, wobei ein Mo-Bi-Fe-Cr-O- oder Mo-Bi-Fe-Zr-O-haltiges Multimetalloxidsystem besonders bevorzugt ist. Bevorzugte Systeme sind beispielsweise beschrieben in US 4,547,615 (Mo₁₂BiFe₀,₁Ni₈ZrCr₃K₀,₂Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co₄,₅Ni_{2,5}Cr_{0,5}K_{0,1}Oₓ, Mo₁₃,₇₅BiFe₃Co₄,₅Ni₂,₅Ge₀,₅K₀,₈Oₓ, Mo₁₂BiFe₃Co_{4,5}Ni₂,₅Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co₄,₅Ni_{2,5}Sn_{0,5}K_{0,1}Oₓ), DE-A 25 30 959 und DE-A 24 47 825 (Mo₁₂BiFe₃Co_{4,5}Ni_{2,5}W₀,₅K_{0,1}Oₓ). Herstellung und Charakterisierung der genannten Katalysatoren sind ausführlich in den zitierten Schriften beschrieben.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als sogenannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Insbesondere hat es sich als günstig erwiesen, den für die Oxidehydrierung der n-Butene zu Butadien bevorzugt eingesetzten Mo-Bi-Fe-O-haltigen Katalysator als Vollkatalysator einzusetzen. Des Weiteren ist es möglich, die Aktivmassen der Katalysatoren auf einen Träger, beispielsweise einen anorganischen, oxidischen Formkörper, aufzubringen. Derartige Katalysatoren werden in der Regel als Schalenkatalysatoren bezeichnet.

Die Oxidehydrierung wird im Allgemeinen bei einer Temperatur von 220 bis 490 °C, bevorzugt von 250 bis 450 °C und besonders bevorzugt von 300 bis 350 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Durch die Kopplung der nicht-oxidativen katalytischen, bevorzugt autothermen Dehydrierung mit der oxidativen Dehydrierung der gebildeten n-Butene wird eine sehr viel höhere Ausbeute an Butadien, bezogen auf eingesetztes n-Butan, erhalten. Ferner kann die nicht-oxidative Dehydrierung schonender betrieben werden. Vergleichbare Butadien-Ausbeuten wären mit einer ausschließlich nicht-oxidativen Dehydrierung nur zum Preis deutlich niedrigerer Selektivitäten zu erreichen. Mit einer ausschließlich oxidativen Dehydrierung würden nur geringe n-Butan-Umsätze erzielt werden.

Der die oxidative Dehydrierung verlassende Produktgasstrom c enthält neben Butadien und nicht umgesetztem n-Butan noch 2-Buten und Wasserdampf. Als Nebenbestandteile enthält er im Allgemeinen Kohlenmonoxid, Kohlendioxid, Sauerstoff, Stickstoff, Methan, Ethan, Ethen, Propan und Propen, gegebenenfalls Wasserstoff sowie sauerstoffhaltige Kohlenwasserstoffe, sogenannte Oxygenate. Im Allgemeinen enthält er nur noch geringe Anteile an 1-Buten.

Im Allgemeinen weist der die oxidative Dehydrierung verlassende Produktgasstrom c 1 bis 40 Vol.-% Butadien, 20 bis 80 Vol.-% n-Butan, 0,5 bis 40 Vol.-% 2-Buten, 0 bis 5 Vol.-% 1-Buten, 0 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 70 Vol.-% Stickstoff, 0 bis 10 Vol.-% Kohlenstoffoxide und 0 bis 10 Vol.-% Oxygenate auf. Oxygenate können beispielsweise Furan, Essigsäure, Maleinsäureanhydrid, Ameisensäure und Butyraldehyd sein.

In einem Verfahrensteil D werden die von den C₄-Kohlenwasserstoffen (n-Butan, isoButan, 1-Buten, cis-/trans-2-Buten, iso-Buten, Butadien) verschiedenen leicht siedenden Nebenbestandteile zumindest teilweise, vorzugsweise aber im wesentlichen vollständig aus dem Produktgasstrom der n-Butan-Dehydrierung abgetrennt, wobei ein C₄-Produktgasstrom d erhalten wird.

In einer Ausführungsform des erfindungsgemäßen Verfahrens wird im Verfahrensteil D zunächst Wasser aus dem Produktgasstrom c abgetrennt. Die Abtrennung von Wasser kann beispielsweise durch Auskondensieren durch Abkühlen und/oder Verdichten des Produktgasstroms c erfolgen und kann in einer oder mehreren Abkühlungs- und/oder Verdichtungsstufen durchgeführt werden.

Die Abtrennung der leicht siedenden Nebenbestandteile aus dem Produktgasstrom kann durch übliche Trennverfahren wie Destillation, Rektifikation, Membranverfahren, Absorption oder Adsorption erfolgen.

Zur Abtrennung des im Produktgasstrom c enthaltenen Wasserstoffs kann das Produktgasgemisch, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

Das in dem Produktgasstrom c enthaltene Kohlendioxid kann durch CO₂-Gaswäsch abgetrennt werden. Der Kohlendioxid-Gaswäsche kann eine gesonderte Verbrennungsstufe vorgeschaltet werden, in der Kohlenmonoxid selektiv zu Kohlendioxid oxidiert wird.

In einer bevorzugten Ausführungsform des erfindungsgemäßen Verfahrens werden die nicht kondensierbaren oder leicht siedenden Gasbestandteile wie Wasserstoff, Sauerstoff, Kohlenstoffoxide, die leicht siedenden Kohlenwasserstoffe (Methan, Ethan, Ethen, Propan, Propen) und gegebenenfalls Stickstoff in einem Absorptions/Desorptions-Cyclus mittels eines hoch siedenden Absorptionsmittels abgetrennt, wobei ein C₄-Produktgasstrom c erhalten wird, der im Wesentlichen aus den C₄-Kohlenwasserstoffen besteht. Im Allgemeinen besteht der C₄-Produktgasstrom c zu mindestens 80 Vol.-%, bevorzugt zu mindestens 90 Vol.-%, besonders bevorzugt zu mindestens 95 Vol.-% aus den C₄-Kohlenwasserstoffen. Im Wesentlichen besteht der Strom d aus n-Butan, 2-Buten und Butadien.

Dazu wird in einer Absorptionsstufe der Produktgasstrom c nach vorheriger Wasserabtrennung mit einem inerten Absorptionsmittel in Kontakt gebracht und werden die C₄-Kohlenwasserstoffe in dem inerten Absorptionsmittel absorbiert, wobei mit C₄-Kohlenwasserstoffen beladenes Absorptionsmittel und ein die übrigen Gasbestandteile enthaltendes Abgas erhalten werden. In einer Desorptionsstufe werden die C₄-Kohlenwasserstoffe aus dem Absorptionsmittel wieder freigesetzt.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Die Absorption kann durch einfaches Durchleiten des Produktgasstroms c durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken―, Zentrifugal― und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht― und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure―n―butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.―%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption der C₄-Kohlenwasserstoffe wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

In einer Verfahrensvariante wird der Desorptionsschritt durch Entspannung und/oder Erhitzen des beladenen Desorptionsmittels durchgeführt.

Die Abtrennung D ist im Allgemeinen nicht ganz vollständig, so dass in dem C₄-Produktgasstrom - je nach Art der Abtrennung - noch geringe Mengen oder auch nur Spuren der weiteren Gasbestandteile, insbesondere der leicht siedenden Kohlenwasserstoffe, vorliegen können.

In einem Verfahrensteil E wird der C₄-Produktgasstrom d in eine Destillationszone eingespeist und in einen Wertproduktstrom e1 aus einem Butadien/Butan-Azeotrop und einen Strom e2, der im Wesentlichen aus n-Butan und 2-Buten besteht, aufgetrennt.

Die Destillationszone besteht im Allgemeinen aus einer Destillationskolonne mit im Allgemeinen 30 bis 80, bevorzugt 40 bis 75 theoretischen Trennstufen. Geeignet sind beispielsweise Glockenbodenkolonnen, Füllkörperkolonnen, Packungskolonnen oder Trennwandkolonnen. Das Rücklaufverhältnis beträgt im Allgemeinen 10 bis 50. Die Destillation wird im Allgemeinen bei einem Druck von 5 bis 20 bar durchgeführt.

Im oberen Teil der Kolonne, vorzugsweise am Kolonnenkopf, wird ein Butadien/n-Butan-Gemisch e1 abgezogen. Das Butadien/n-Butan-Gemisch kann die Zusammensetzung des Azeotrops aufweisen oder einen geringeren Butadiengehalt aufweisen, im Allgemeinen enthält das Butadien/n-Butan-Gemisch mindestens 60 Vol.-% Butadien.

Im unteren Teil der Kolonne, vorzugsweise im unteren Fünftel der Kolonne, besonders bevorzugt am Kolonnensumpf bis maximal 5 theoretische Böden oberhalb des Kolonnensumpfs, wird ein im Wesentlichen aus n-Butan und 2-Buten bestehender Strom e2 abgezogen. Daneben kann der Sumpfabzugsstrom noch Butadien enthalten. Im Allgemeinen enthält der Strom e2 80 bis 100 Vol.-% n-Butan und 0 bis 20 Vol.-% 2-Buten und Butadien. Die Angabe der Vol.-% bezieht sich auf das gasförmige Gemisch.

Von dem Strom e2 kann ein Purgegasstrom abgetrennt werden, um die Aufpegelung von Hochsiedern zu vermeiden. Der Strom e2 wird in die erste Dehydrierzone zurückgeführt.

Das Butadien/n-Butan-Gemisch ohne weitere Auftrennung in der Hydrocyanierung von Butadien zu Adipodinitril eingesetzt werden.

### Beispiel

Ein n-Butan enthaltender Einsatzgasstrom (4), welcher durch Vereinigung eines Frischgasstroms (1) und eines Rückführstroms (12) erhalten wird, wird der ersten, autotherm betriebenen, nicht-oxidativen katalytischen n-Butan-Dehydrierungsstufe (BDH) zugeführt. Zur Bereitstellung der für die endotherme Dehydrierung notwendigen Wärme wird selektiv Wasserstoff verbrannt, der zusammen mit der Verbrennungsluft als Strom (2) zugeführt wird. Um einer Verkokung des Katalysators entgegenzuwirken und die Standzeit des Katalysators zu verlängern wird außerdem Wasserdampf (3) zugegeben. Es wird ein Dehydriergasgemisch (5) erhalten, das nach Austritt aus der BDH abgekühlt und der zweiten, oxidativen n-Butan-Dehydrierstufe (ODH) zugeführt. Der ODH wird ferner ein Luftstrom (6) zugeführt. Für BDH und ODH wurden basierend auf experimentellen Ergebnissen die in Tabelle 1 wiedergegebenen Umsatzgrade bzw. Selektivitäten angenommen.

**Tabelle 1**

| Reaktionsstufe | Umsatz [%] | Selektivität [%] |
|---|---|---|
| Autotherme | 50,5 | 98,4 |
| Dehydrierung (BDH) | (n-Butan) | (zu Butenen/Butadien) |
| Oxidative | 100,0 (1-Buten) | 95,0 |
| Dehydrierung (ODH) | 92,7(2-Buten) | (zu Butadien) |

Das Austrittsgas (7) der ODH wird mehrstufig mit Zwischenkühlung verdichtet. Das bei den Zwischenkühlungen anfallende wässrige Kondensat (8) wird aus dem Prozess ausgeschleust. Das komprimierte butadienhaltige Gas (9) wird einer Absorptionsstufe zugeführt, die mit Tetradecan als Absorptionsmittel betrieben wird. In der Absorptionsstufe wird ein Inertgasstrom (10) von dem C₄-Kohlenwasserstoffstrom (11) abgetrennt. Der C₄-Kohlenwasserstoffstrom (11) wird in einer Destillationsstufe in einen Butadien reichen Strom (13) und einen im Wesentlichen aus n-Butan bestehenden Rückführstrom (12) getrennt.

Die Ergebnisse der Simulation sind in Tabelle 2 wiedergegeben. Die Zusammensetzung der Stoffströme (1) bis (13) ist in Volumenanteilen angegeben.

**Tabelle 2**

| Strom-Nr. | 1 | 2 | 3 | 4 | 5 | 6 | 7 | 8 | 9 | 10 | 11 | 12 | 13 |
|---|---|---|---|---|---|---|---|---|---|---|---|---|---|
| Menge [kg/h] | 28904 | 18977 | 1180 | 50051 | 70209 | 70834 | 141043 | 13456 | 127587 | 79734 | 47852 | 21147 | 26705 |
| Propan | 0,0000 | 0,0000 | 0,0000 | 0,0157 | 0,0095 | 0,0000 | 0,0042 | 0,0019 | 0,0047 | 0,0001 | 0,0213 | 0,0362 | 0,0100 |
| Propen | 0,0000 | 0,0000 | 0,0000 | 0,0000 | 0,0000 | 0,0000 | 0,0000 | 0,0000 | 0,0000 | 0,0000 | 0,0000 | 0,0000 | 0,0000 |
| Butan | 1,0000 | 0,0000 | 00000 | 0,9374 | 0,1894 | 0,0000 | 0,0847 | 0,0000 | 0,1000 | 0,0004 | 0,4590 | 0,8555 | 0,1600 |
| 1-Buten | 0,0000 | 0,0000 | 0,0000 | 0,0000 | 0,0597 | 0,0000 | 0,0000 | 0,0000 | 0,0000 | 0,0000 | 0,0000 | 0,0000 | 0,0000 |
| 2-Buten | 0,0000 | 0,0000 | 0,0000 | 0,0183 | 0,1293 | 0,0000 | 0,0042 | 0,0056 | 0,0040 | 0,0000 | 0,0182 | 0,0422 | 0,0000 |
| 1,3-Butadien | 0,0000 | 0,0000 | 0,0000 | 0,0087 | 0,0119 | 0,0000 | 0,0816 | 0,0000 | 0,0964 | 0,0004 | 0,4422 | 0,0200 | 0,7605 |
| Wasser | 0,0000 | 0,0000 | 1,0000 | 0,0200 | 0,1513 | 0,0000 | 0,1600 | 0,9908 | 0,0103 | 0,0018 | 0,0410 | 0,0461 | 0,0371 |
| Kohlendioxid | 0,0000 | 0,0000 | 0,0000 | 0,0000 | 0,0031 | 0,0000 | 0,0174 | 0,0013 | 0,0204 | 0,0212 | 0,0172 | 0,0000 | 0,0302 |
| Wasserstoff | 0,0000 | 0,2853 | 0,0000 | 0,0000 | 0,2074 | 0,0000 | 0,0928 | 0,0000 | 0,1095 | 0,1399 | 0,0000 | 0,0000 | 0,0000 |
| Sauerstoff | 0,0000 | 0,1429 | 0,0000 | 0,0000 | 0,0001 | 0,2100 | 0,0450 | 0,0001 | 0,0531 | 0,0676 | 0,0008 | 0,0000 | 0,0014 |
| N2 | 0,0000 | 0,5718 | 0,0000 | 0,0000 | 0,2383 | 0,7900 | 0,5099 | 0,0002 | 0,6018 | 0,7686 | 0,0004 | 0,0000 | 0,0007 |
| Temperatur [°C] | 25,0 | 25,0 | 150,0 | 500,0 | 550,0 | 115,0 | 400,0 | 55,0 | 55,0 | 35,0 | 81,5 | 87,0 | 79,0 |
| Druck [bar] | 3,1 | 3,1 | 3,1 | 3,1 | 2,7 | 2,7 | 2,4 | 15,0 | 15,0 | 15,0 | 12,0 | 12,0 | 12,0 |

## Patentansprüche

1. Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative katalytische Dehydrierung von n-Butan, wobei ein Produktgasstrom b enthaltend n-Butan, 1-Buten, 2-Buten, Butadien, Wasserstoff, leichtsiedende Nebenbestandteile und gegebenenfalls Wasserdampf erhalten wird;
C) Einspeisung des Produktgasstroms b der nicht-oxidativen katalytischen Dehydrierung und eines sauerstoffhaltigen Gases in mindestens eine zweite Dehydrierzone und oxidative Dehydrierung von 1-Buten und 2-Buten, wobei ein Produktgasstrom c enthaltend n-Butan, 2-Buten, Butadien, Wasserstoff, leichtsiedende Nebenbestandteile und Wasserdampf erhalten wird, welcher einen höheren Gehalt an Butadien als der Produktgasstrom b aufweist;
D) Abtrennung von Wasserstoff, der leichtsiedenden Nebenbestandteile und von Wasserdampf, wobei ein C₄-Produktgasstrom d im Wesentlichen bestehend aus n-Butan, 2-Buten und Butadien erhalten wird;
E) Einspeisung des C₄-Produktgasstroms d in eine Destillationszone und Abtrennung eines Butadien/Butan-Gemischs als Wertproduktstrom e1, wobei ein Strom e2 im Wesentlichen bestehend aus n-Butan und 2-Buten verbleibt.
F) Rückführung des Stroms e2 in die erste Dehydrierzone.

2. Verfahren nach Anspruch 1, **dadurch gekennzeichnet, dass** die nicht-oxidative katalytische Hydrierung von n-Butan autotherm durchgeführt wird.

3. Verfahren nach Anspruch 1 oder 2, **dadurch gekennzeichnet, dass** der n-Butan enthaltende Einsatzgasstrom aus liquefied petroleum gas (LPG) gewonnen wird.

## Claims

1. A process for preparing butadiene from n-butane having the steps of
A) providing a feed gas stream a comprising n-butane;
B) feeding the feed gas stream a comprising n-butane into at least one first dehydrogenation zone and nonoxidatively catalytically dehydrogenating n-butane to obtain a product gas stream b comprising n-butane, 1-butene, 2-butene, butadiene, hydrogen, low-boiling secondary constituents and in some cases steam;
C) feeding the product gas stream b of the nonoxidative catalytic dehydrogenation and an oxygenous gas into at least one second dehydrogenation zone and oxidatively dehydrogenating 1-butene and 2-butene to obtain a product gas stream c comprising n-butane, 2-butene, butadiene, hydrogen, low-boiling secondary constituents and steam, said product gas stream c having a higher content of butadiene than the product gas stream b;
D) removing hydrogen, the low-boiling secondary constituents and steam to obtain a C₄ product gas stream d substantially consisting of n-butane, 2-butene and butadiene;
E) feeding the C₄ product gas stream d into a distillation zone and removing a butadiene/butane mixture as the product of value stream e1, to leave a stream e2 consisting substantially of n-butane and 2-butene;
F) recycling the stream e2 into the first dehydrogenation zone.

2. The process according to claim 1, wherein the nonoxidative catalytic hydrogenation of n-butane is carried out autothermally.

3. The process according to claim 1 or 2, wherein the feed gas stream comprising n-butane is obtained from liquefied petroleum gas (LPG).

## Revendications

1. Procédé de fabrication de butadiène à partir de n-butane, comprenant les étapes suivantes :
A) on prépare un courant de gaz d'utilisation a contenant du n-butane ;
B) on injecte le courant de gaz d'utilisation a contenant du n-butane dans au moins une première zone de déshydrogénation, on effectue une déshydrogénation catalytique non oxydante du n-butane, un courant de gaz de produits b, contenant du n-butane, du 1-butène, du 2-butène, du butadiène, de l'hydrogène, des composants secondaires à bas point d'ébullition et, éventuellement, de la vapeur d'eau étant obtenu;
C) on injecte le courant de gaz de produits b provenant de l'étape de déshydrogénation catalytique non oxydante et un gaz contenant de l'oxygène dans au moins une seconde zone de déshydrogénation et on effectue une déshydrogénation oxydante du 1-butène et du 2-butène, un courant de gaz de produits c contenant du n-butane, du 2-butène, du butadiène, de l'hydrogène, des composants secondaires à bas point d'ébullition et de la vapeur d'eau étant obtenu, lequel présente une teneur en butadiène supérieure à celle du courant de gaz de produits b ;
D) on sépare l'hydrogène, les composants secondaires à bas point d'ébullition et la vapeur d'eau, un courant de gaz de produits en C₄ d constitué sensiblement de n-butane, de 2-butène et de butadiène étant obtenu;
E) on injecte le courant de gaz de produits en C₄ d dans une zone de distillation et on sépare un mélange de butadiène/butane sous la forme d'un courant de produits intéressants e1, de sorte qu'il reste un courant e2 constitué sensiblement de n-butane et de 2-butène ;
F) on renvoie le courant e2 dans la première zone de déshydrogénation.

2. Procédé selon la revendication 1, **caractérisé en ce que** l'hydrogénation catalytique non oxydante du n-butane se fait de manière autothermique.

3. Procédé selon les revendications 1 ou 2, **caractérisé en ce que** le courant de gaz d'utilisation contenant du n-butane provient de gaz de pétrole liquéfié (GPL).
